# EUROPEAN PATENT SPECIFICATION

(11) **EP 3 320 928 B1**
(45) Date of publication and mention of the grant of the patent: **26.08.2020**
(21) Application number: 16821531.7
(22) Date of filing: 17.05.2016
(51) Int. Cl.: A61M 5/168, A61M 5/148, A61M 5/14, A61M 5/142, A61M 39/24

(54) **DRUG INJECTION DEVICE**
ARZNEIMITTELINJEKTIONSVORRICHTUNG
DISPOSITIF D'INJECTION DE MÉDICAMENTS

(30) Priority: 08.07.2015 KR 20150097270
(43) Date of publication of application: 16.05.2018
(73) Proprietor: Cebika Inc., Uiwang-si, Gyeonggi-do 16006 (KR)
(72) Inventor: CHO, Byoung Chic, Seongnam-si Gyeonggi-do 13582 (KR)
(74) Representative: Hansen, Norbert
(86) International application number: PCT/KR2016/005212
(87) International publication number: WO 2017/007125

(56) References cited:
- KR-A- 20040 014 543
- KR-B1- 100 749 266
- KR-B1- 101 066 819
- US-A- 456 558
- US-A- 6 056 727
- US-A1- 2006 004 330
- US-A1- 2013 184 576
- US-B2- 7 335 186

## Description

### Technical Field

The present invention relates to a medicine infusing apparatus, and more particularly, to a medicine infusing apparatus that may conveniently and safely infuse a medicine according to a usage of the medicine with a compact configuration.

### Background Art

In the medical field, medicines injected or infused into the patients naturally have to be injected according to the usages of the medicines, and among the usages, an amount of the medicine, an infusion speed, and an infusion time are very important factors.

In particular, injection of a pain killer that is necessary to manage pain accompanied after a surgery of the patient is very important, adjustment of pain after the surgery is one scale for determining a result of a surgery, and generally a time period for which the pain is most severe after the surgery is 72 hours from the surgery. The pain treatment after the surgery is a problem that has to be solved in all surgeries.

Accordingly, in 1995, U.S. pain society defined pain as a fifth vital sign.

Main medicines user to adjust pain include a muscle relaxant, a sedative, and a pain killer. The most general method used to adjust pin is to intermittently inject the medicine whenever the patient appeals pain.

US 2006/004330 A1 describes a device for control of fluid flow with a closed loop quasi-static adjustment of an in-line pressure-based resistance.

US 2013/184576 A1 describes a valve-equipped hand switch and a chemical liquid introduction system, wherein a contrast medium in a syringe is delivered to a patient line through a first port, a main passage around a spacer portion, and a second port.

US 456 558 A describes an electrode for secondary batteries, which electrode consists of a conducting-plate containing active material.

However, according to the intermittent injection method, pain cannot be uniformly adjusted, and as a small amount of medicine has to be frequently infused, an excessive amount of medicine is injected so that restraint of respiration, an excessive sedation, a lung/intestine problem such as nausea-vomiting, a urinary retention, or a pruritus may be caused according to the kind of the medicine and in a severe case, drug addiction may be caused.

Accordingly, a patient-controlled analgesia (PCA) using a PCA apparatus that allows the patient to infuse a drug by himself or herself instead of adjusting pain through the intermittent infusing method has been developed.

The PCA method was developed and practiced by Sechzer, and is currently being used in various areas. The PCA method employs a PCA infuser system (or a PCA pump) that is a PCA apparatus through which the patient may directly adjust injection of a medicine while continuously infusing the medicine that shows low side-effect rate and high effect.

The combination of medicines applied to the PCA, an initial loading dose, a background infusion rate (a basal rate), a demand dose (a bolus dose), a lockout interval, and a combination of used medicines are not standardized yet, and various method are used.

The initial loading does is a dose of a medicine that is injected when a PCA system is initially applied after a surgery, and a demand dose is a set dose of a medicine that is additionally injected when the patient feels pain. The lockout interval is a time interval for which injection of a medicine is interrupted until a demand dose of medicine is injected again after the demand dose of medicine is injected, and is a time period that is set to prevent infusion of an excessive amount of medicine. The background infusion rate is an amount of medicine that is injected at a specific speed regardless of the demand dose.

Although the frequency and degree of the side-effects of the pain treatment method by the PCA are lower than those of the pain treatment method by the intermittent infusion, it is important to select a medicine and determine a background infusion rate and a demand dose according to the state of the patient because it may also cause side-effects, and in particular, it is very important to manage a lockout interval after injection of a demand dose.

Accordingly, in recent years, the PCA method is carried out by installing a pump in an electronic control apparatus to control various factors of the PCA method, but it is difficult to promptly and flexibly cope with the large-scale of the apparatus and the state of the patient.

As an example, Korean Patent Application Publication No. 201-0005182 discloses an infusion apparatus used for a PCA method, but the infusion apparatus performs a control to restrain generation of bubbles that may be generated when a demand dose of medicine is infused and to prevent additional introduction of a medicine when a stored demand dose of medicine is infused and the efficient control of the factors of the PCA method is still a problem that is to be solved.

### (Patent Document 1) KR 2014-0005182 A

### Disclosure

### Technical Problem

Accordingly, the present invention has been made in an effort to solve the above-mentioned problems, and provides an apparatus that may conveniently and promptly infuse a medicine injected to the patient according to the usage of the medicine and may prevent a danger of side-effects due to continuous infusion by certainly securing an interruption time.

### Technical Solution

The invention is defined according to claim 1.

The regulation part may include a locking plate located on another side of the cover to be rotatable, and a stopping pin extending from one side surface of the locking plate toward one side of the cover, the press part may include a stopping part extending from one side thereof toward one side of the cover), and the movement of the press part may be regulated while the stopping pin is coupled to or separated from the stopping part according to a rotational direction of the locking plate.

The locking plate may include a seating part located at a central portion thereof, and a locking pin located to be inserted into and extracted from the seating part.

The rotation of the locking plate may be regulated according to a location of the locking pin.

Rotation of the locking plate may be prevented if the locking pin is located inside the seating part, and rotation of the locking plate may be allowed if the locking pin is located outside the seating part.

The medicine infusing apparatus may further include a release member coupled to the regulation part such that an operation of the regulation part is performed.

The medicine infusing apparatus may further include a release member including a release boss that is inserted into a through-hole formed in the seating part, the release member may be coupled to the regulation part, and the locking pin may be located outside the seating part if the release boss passes through the through-hole to press the locking pin, and the locking pin may be located inside the seating part if the release member is released from the regulation part.

The first tube and the second tube may be located in the cover to be linearly parallel to each other.

The medicine infusing apparatus may further include a flow rate adjusting apparatus installed in one or more passages of the first tube and the second tube.

The medicine infusing apparatus includes a check valve installed on a discharge direction of the second tube.

The medicine infusing apparatus may further include a fastening part located on one side of the cover.

### Advantageous Effects

As described above, according to the medicine infusing apparatus according to the present invention, the apparatus may be conveniently used even only by connecting the apparatus to a medicine supply unit with a compact configuration and may promptly infuse a medicine according to the state of the patient by conveniently manipulating the apparatus.

Further, a danger of the infusion of a medicine due to the usage of the medicine may be effectively prevented.

### Description of the Invention

Fig. 1 is a perspective view of a medicine infusing apparatus according to an embodiment of the present invention;
Fig. 2 is an exploded perspective view of the medicine infusing apparatus according to the embodiment of the present invention;
Fig. 3 is an exploded perspective view of tubes 110 and 120 and a storage part 250 that are configurations of the medicine infusing apparatus according to the embodiment of the present invention;
Fig. 4 illustrates perspective views of a press part that is a configuration of the medicine infusing apparatus according to the embodiment of the present invention, and illustrates perspective views viewed from the top (Fig. 4A) and the bottom (Fig. 4B);
Fig. 5 illustrates a plan view and a bottom view obtained by viewing one side of a lower cover 120 that is a configuration of the medicine infusing apparatus according to the embodiment of the present invention from the top (Fig. 4A) and the bottom (Fig. 4B);
Fig. 6 illustrates a plan view and a bottom view obtained by viewing one side of a locking plate 410 of a regulation part 400 that is a configuration of the medicine infusing apparatus according to the embodiment of the present invention from the top (Fig. 4A) and the bottom (Fig. 4B);
Fig. 7 is a sectional view taken along a line A-A' of Fig. 1; and
Fig. 8 is a sectional view taken along a line B-B' of Fig. 1.

### Best Mode

The objectives, features, and other advantages of the present invention will be more apparent by describing exemplary embodiments of the present invention in detail with reference to the accompanying drawings. It should be noted that the drawings are not to precise scale and may be exaggerated in thickness of lines or size of components for descriptive convenience and clarity. In addition, terms used herein are defined by taking functions of the present invention into account and can be changed according to user or operator custom or intention. Therefore, definition of the terms should be made according to the overall disclosure set forth herein.

Further, the embodiments are provided exemplarily for description of the present invention, and are not intended to limit the technical scope of the present invention.

The elements constituting the medicine infusing apparatus according to the present invention may be integrally used or separately used as occasion demands. Further, some elements may be omitted according to the usage of the apparatus.

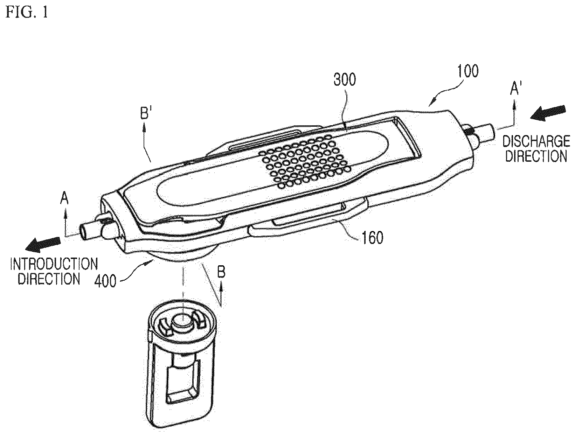

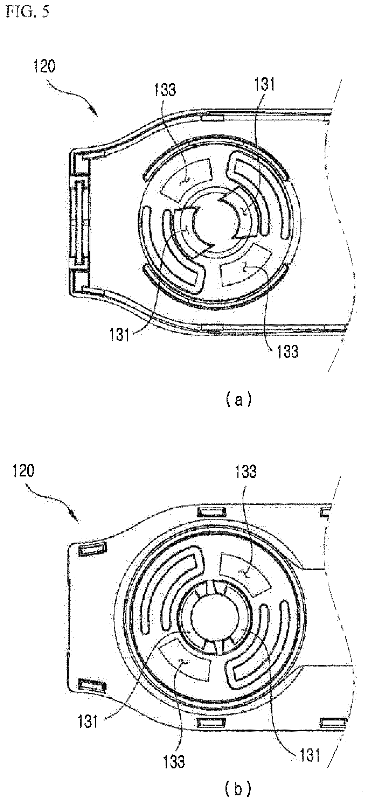

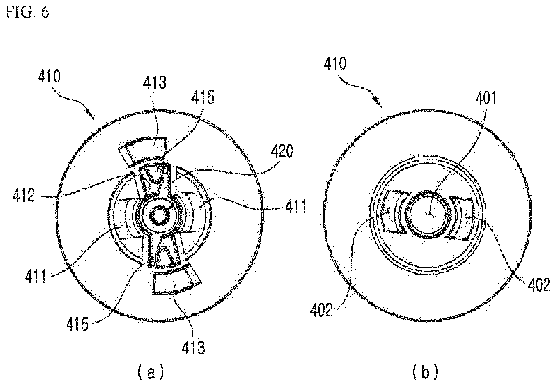

Hereinafter, the medicine infusing apparatus according to an embodiment of the present invention will be described with reference to FIGS. 1 to 8.

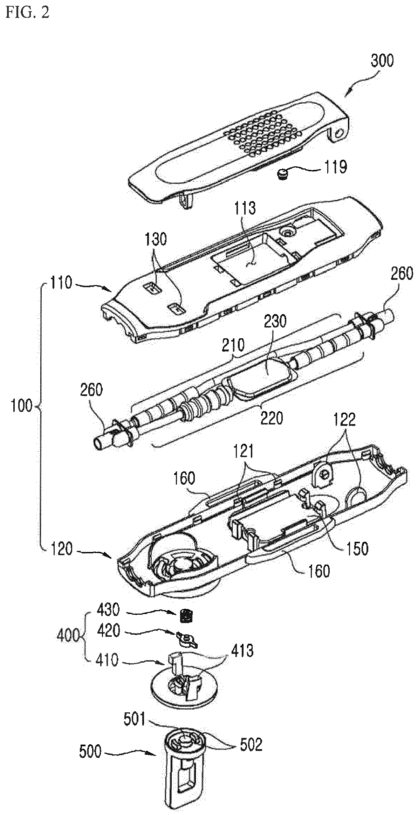

The medicine infusing apparatus according to the embodiment of the present invention, as illustrated in Figs. 1 and 2, may include a cover 100, first and second tubes 210 and 220, a storage part 250, a press part 300, a regulation part 400, and a release member 500.

The cover forms an external appearance of the medicine infusing apparatus, and includes an upper cover 110 and a lower cover 120, a plurality of coupling recesses 111 formed along an outer peripheral surface of the upper cover 110 and a plurality of coupling bosses 121 formed along an inner peripheral surface of the lower cover 120 are coupled to each other through snap coupling to form a specific space in the interior thereof, and additionally, may be coupled by a cover fixing member 119 that passes through one side of the upper cover 110 to be connected to an inside of the lower cover 120.

The first tube 210 is located along a lengthwise direction of the cover 100 from an inside of the cover 100, and a first medicine is introduced through one side of the first tube 210 and is discharged to an opposite side.

The second tube 220 is located along a lengthwise direction of the cover 100 from an inside of the cover 100.

Opposite sides of the storage part 250 are connected to the second tube 220 to be located inside the cover 100, and preferably, are supported by a storage part support body 150 provided inside the lower cover 120 to be fixed inside the cover 100.

That is, the first tube 210 and the second tube 220 are located in parallel to each other along a lengthwise direction of the cover 100, and preferably, opposite ends of the tubes 210 and 220 are connected to branch parts of a Y-connection pipe 260 so that the tubes 210 and 220 may be disposed in parallel to each other inside the cover 100.

A specific space is provided in the interior of the storage part 250 such that a second medicine may be accommodated in the specific space, and the accommodated second medicine is discharged from the storage part 250 by a pressure applied by the storage part 250 to be discharged through the second tube 220.

The first medicine and the second medicine are not limited, and as an example, the first medicine may be a medicine that requires continuous injection and may be a pain adjusting medicine injected according to the infusion rate, and the second medicine may be a pain adjusting medicine an amount of which corresponds to a dose, which is infused once, according to a preset demand dose amount that is additionally injected when the patient feels pain.

In the medicine infusing apparatus, the storage part 250 in which the second medicine is stored is generally far away from the patient for control thereof and disposed individually so that it is often difficult to promptly cope with a situation of the patient, and the medicine infusing apparatus according to the present invention includes a first tube 210, a second tube 220, and a storage part 250 in the interior of a compact cover to be conveniently used and controlled even only by connecting the tubes 210 and 220 to a medicine supply unit (not illustrated), and preferably, a member such as a belt is fastened to a fastening part 160 located on one side of the cover 100 such that the medicine infusing apparatus may be conveniently attached to the body of the patient.

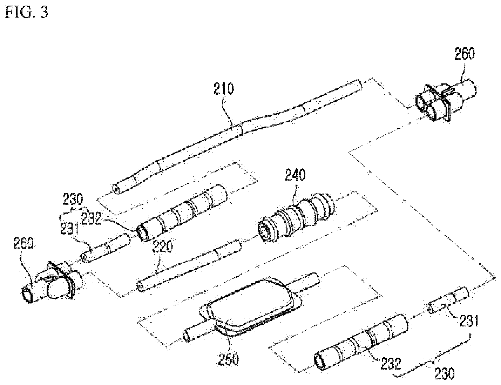

Meanwhile, as illustrated in Fig. 3, flow rage adjusting apparatuses 230 for adjusting the flow rates of the flowing medicines are provided on the passages of the tubes 210 and 220. As illustrated, the flow rate adjusting apparatus 230 includes a glass tube 231 connected to sides of the tube 210 and 220, and a support that facilitates connections of the glass tube 231 and the tube 210 and 220 and surrounds a circumference of the glass tube 231 to support the glass tube 231. By adjusting the inner diameter of the glass tube 231, the flow rates of the medicine flowing through the tubes 210 and 220 may be adjusted.

A check valve 240 is installed on one side of the storage part 250 of the second tube 220, in detail, in a discharge direction of the second medicine discharged from the storage part 250 so that the medicine introduced into the storage part 250 through an inlet side of the second tube 220 is not discharged to an outlet side before it is fully filled in the storage part 250 to prevent the medicine from being injected to the patient and to allow the second medicine to be discharged from the storage part 250 and discharged through the second tube 220 only when a specific pressure is applied to the storage part 250 even after the storage part 250 is fully filled with the second medicine.

The press part 300 is located on one side of the cover to be movable, and may apply or release a pressure to the storage part 250 located inside the cover 100 through movement thereof. Any means capable of applying a pressure from one side of the cover 100 such that the second medicine accommodated in the storage part 250 may be discharged is sufficient as a configuration of the press part 300.

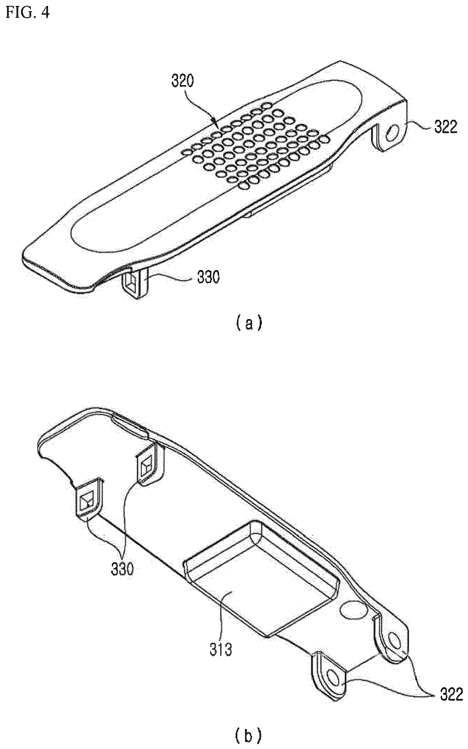

In detail, as illustrated in Figs. 2 and 4, a hinge opening 322 located on one side of the upper cover 110 passes through the upper cover 110 to be coupled to a hinge member 122 located on one side of the interior of the lower cover 120 so that the upper cover 110 may be hinged about the coupling part, and a protruding press member 313 is located on a surface of the press part 300 that is close to the cover 100 so that the press member 313 may apply or release a pressure to the storage part 250 by a hinge movement of the press part 300 through a press window 113 opened on the press part 300 of the cover 100.

Additionally, a medicine display window 320 formed of a transparent material may be provided on one side of the press part 300 so that the second medicine accommodated in the storage part 250 may be identified through the press window 113.

Accordingly, the user may promptly discharge and infuse the second medicine with a simple operation of pressing the press part 300 with a hand. Meanwhile, when the usage of the second medicine is particular according to the kind of the second medicine or a side effect or a danger is caused by the injection timing, the simple infusion of the second medicine may be dangerous, and the regulation part 400 is provided as a means for regulating the infusion.

The regulation part 400 is a means that is located on one side of the lower cover 120 and capable of regulating movement of the press part 300 by providing a regulation means capable of regulating movement of the press part 300 in the lower cover 120.

In detail, the regulation part 400 may include a locking plate 410, a stopping pin 413, and a locking pin 420.

As illustrated in Figs. 2, 5, and 6, the locking plate 410 is coupled to be rotatable through the coupling member 411 located around a central axis thereof and a coupling recess 131 located on one side of the lower cover 120, preferably, through snap coupling.

A seating part 412 having a specific space is provided around the central axis of the locking plate 410, in detail, inside the coupling member 411, a locking pin 420 that is inserted into and extracted from the seating part 412 is provided, the locking pin 420 is fixed not to be rotated, and an elastic member 430 that elastically support the locking pin 420 may be located between the locking pin 420 and the lower cover 120.

Regulation bosses 415 protruding toward the inside of the seating part 412 may be located at opposite lengthwise ends of the seating part 412, and opposite ends of the locking pin 420 may be located on opposite sides of the regulation bosses 415 as the locking plate 410 is rotated. Further, a through-hole 401 is formed on a lower surface of the seating part 412.

The stopping pin 413 extends from a side surface of the lower cover 120 of the locking plate 410 toward the lower cover 120, and the stopping pin 413 is located inside the cover after passing through the through-hole 133 formed in the lower cover 120 to be moved as the locking plate 410 is rotated so that an operation of the press part 300 may be regulated by preventing or allowing a hinge movement of the press part 300 as the stopping pin 413 extends from a side surface of the upper cover 110 of the press part 300 toward the upper cover 110 to be coupled to or separated from the stopping part 330 that passed through the upper cover 110.

The release member 500 includes a release boss 501 located on one side thereof, and an insertion boss 502 located around the release boss 501.

The locking plate 410 is rotated as the release member 500 is coupled to the regulation part 400 to be rotated.

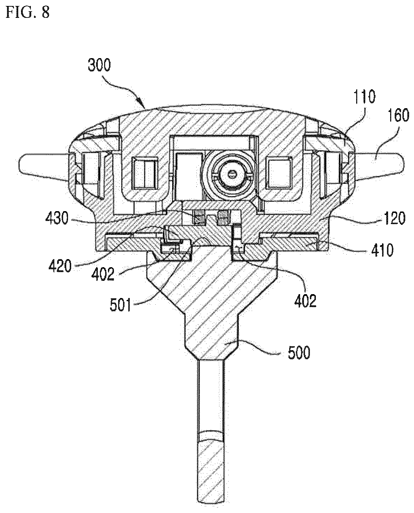

Preferably, the release boss 501 is located at a central portion of one side of the release member 500 so that the locking pin 420 is pressed to be moved to the outside of the seating part 412 as the release boss 501 is inserted into the through-hole formed in the seating part of the locking plate 410 when the regulation part 400 of the release member 500 is coupled (see Fig. 8).

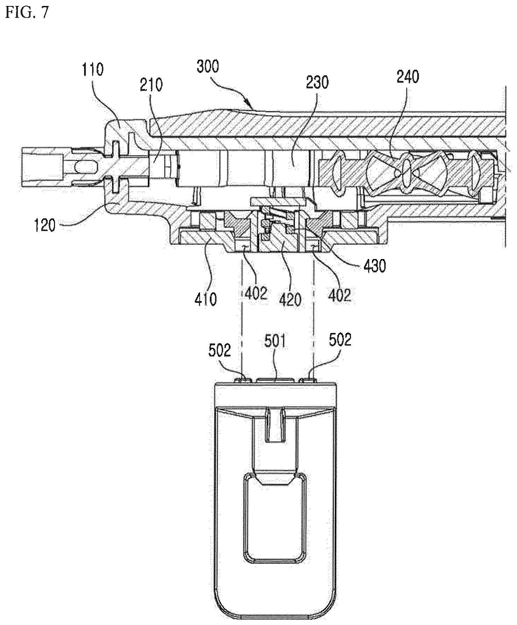

When the regulation part 400 of the release member 500 is coupled, the insertion boss 502 is inserted into a recess located on an opposite side surface of the lower cover 120 of the locking plate 410 so that the locking plate 410 is rotated as the release member 500 is rotated (see Fig. 7).

Next, an operation of the medicine infusing apparatus according to the present invention will be described.

As illustrated in Figs. 1, 7, and 8, the press part 300 is adhered to the upper cover 110, and the first medicine is continuously introduced through the first tube 210 while the movement of the press part 300 is restricted in a state in which the stopping pin 413 is coupled to the stopping part 330. Further, because the storage part 250 continues to be pressed between the press part 300 and the lower cover 120 by the press member 313 located in the press part 300, the second medicine cannot be introduced into the storage part 250 through the second tube 220 to be stored.

Further, in this state, the locking pin 420 is stopped by the regulation bosses located in the seating part to prevent rotation of the locking plate 410. That is, the press part 250 is prevented from being operated by rotating the locking plate 410 by force by using a hand or another tool.

In this state, when it is necessary to infuse the second medicine, the release boss 501 passes through the through-hole formed in the seating part by coupling the regulation part 400 to the release member 500 so that the locking pin 420 is separated to the outside of the seating part by pressing the locking pin 420 as illustrated in Fig. 8.

Accordingly, the locking plate 410 maintained by the locking pin 420 and the regulation bosses is released to be rotated, and the locking plate 410 is rotated as the insertion boss 502 inserted into the recess of the locking plate 410 is moved if the release member 500 is rotated in this state, and accordingly, the stopping pin 413 is moved to be separated from the stopping part 330 and the press part 300 may be hinged upwards and downwards.

Accordingly, the second medicine is introduced through an inlet side of the second tube 220 to be introduced into and stored in the storage part, and the discharge of the second medicine is prevented by the check valve 240 located on an outlet side of the second tube 220 as described above while the second medicine is stored.

After it is identified through the medicine display window 320 that the second medicine is fully filled in the storage part 250, a pressure is applied to the storage part 250 by pressing the press part 300 so that the second medicine accommodated in the storage part 250 exceeds a pass pressure of the check valve 240 and is discharged and infused into the patient.

As described above, an operation of the press part 300 is allowed only by the release member 500 so that the second medicine may be infused. That is, the release member 500 functions as a key.

Accordingly, because the release member 500 is managed by a medical expert (a doctor or a nurse) after the second medicine is infused, the second medicine may be injected and a lockout interval may be safely managed so that a danger due to a misuse of the second medicine may be prevented.

As described above, the medicine infusing apparatus of the present invention may be conveniently used even only by connecting the first tube and the second tube to the medicine supply unit because the first tube, the second tube, and the storage part are provided inside the compact tube.

Further, the second medicine may be promptly discharged and infused through a simple operation of pressing the press part with a hand, and a danger of side effects due to a deficiency of a usage for injection of the second medicine may be effectively prevented.

## Claims

1. A medicine infusing apparatus comprising:
a cover (100) having an upper cover (110) and a lower cover(120);
a first tube (210) located inside the cover (100) and through which a first medicine flows;
a storage part (250) located inside the cover (100) at a first side of the lower cover(120), wherein a space accommodating a second medicine is provided in an interior of the storage part(250),
a second tube (220) connected to opposite sides of the storage part (250), such that the second medicine is introduced through an inlet side of the second tube (220) into the storage part (250) and discharged from the storage part (250) through an outlet of the second tube (220) in a discharge direction;
a press part (300) located on one side of the cover and movable by a hinge movement, wherein the press part (300) is configured to apply a pressure to the storage part (250) to discharge the second medicine as the press part (300) is moved by the hinge movement;
a check valve (240) connected to one side of the storage part (250) in the discharge direction and connected to the outlet side of the second tube (220), wherein the check valve (240) is configured to prevent discharging of the second medicine from the storage part before fully filling the storage part with the second medicine and to allow discharging of the second medicine from the storage part only when a specific pressure is applied to the storage part by the hinge movement of the press part (300) after the storage part (250) is fully filled with the second medicine; and
a regulation part (400) at a second side of the lower cover (120) opposite to the first side of the lower cover (120), wherein the regulation part (400) is configured to regulate movement of the press part (300) by preventing or allowing the hinge movement of the press part (300).

2. The medicine infusing apparatus of claim 1, wherein the regulation part (400) includes a locking plate (410) located at the second side of the lower cover (120) to be rotatable, and a stopping pin (413) extending from one side surface of the locking plate (410) toward the first side of the lower cover (120),
wherein the press part (300) includes a stopping part (330) extending from one side thereof toward the first side of the lower cover (120), and
wherein the movement of the press part (300) is regulated while the stopping pin (413) is coupled to or separated from the stopping part (330) according to a rotational direction of the locking plate (410).

3. The medicine infusing apparatus of claim 2, wherein the locking plate (410) includes a seating part (412) located at a central portion thereof, and a locking pin (420) located to be inserted into and extracted from the seating part (412).

4. The medicine infusing apparatus of claim 3, wherein the rotation of the locking plate (410) is regulated according to a location of the locking pin (420).

5. The medicine infusing apparatus of claim 3, wherein rotation of the locking plate (410) is prevented if the locking pin (420) is located inside the seating part (412), and rotation of the locking plate (410) is allowed if the locking pin (420) is located outside the seating part (412).

6. The medicine infusing apparatus of claim 1, further comprising:
a release member (500) coupled to the regulation part (400) such that an operation of the regulation part (400) is performed.

7. The medicine infusing apparatus of claim 5, further comprising:
a release member (500) including a release boss (501) that is inserted into a through-hole (401) formed in the seating part (412),
wherein the release member (500) is coupled to the regulation part (400), and the locking pin (420) is located outside the seating part (412) if the release boss (501) passes through the through-hole (401) to press the locking pin (420), and
wherein the locking pin (420) is located inside the seating part (412) if the release member (500) is released from the regulation part (400).

8. The medicine infusing apparatus of claim 1, wherein the first tube (210) and the second tube (220) are located in the cover (100) to be linearly parallel to each other.

9. The medicine infusing apparatus of claim 8, further comprising:
a flow rate adjusting apparatus (230) installed in one or more passages of the first tube (210) and the second tube (220).

10. The medicine infusing apparatus of claim 1, further comprising:
a fastening part (160) located on one side of the cover (100).

## Patentansprüche

1. Medikamenteninfusionsvorrichtung, umfassend:
eine Abdeckung (100) mit einer oberen Abdeckung (110) und einer unteren Abdeckung (120);
eine erste Röhre (210), die im Inneren der Abdeckung (100) angeordnet ist und durch die ein erstes Medikament fließt;
einen Bevorratungsteil (250), der im Inneren der Abdeckung (100) an einer ersten Seite der unteren Abdeckung (120) angeordnet ist, wobei ein Raum, der ein zweites Medikament unterbringt, in einem Inneren des Bevorratungsteils (250) vorgesehen ist,
eine zweite Röhre (220), die mit gegenüberliegenden Seiten des Bevorratungsteils (250) derart verbunden ist, dass das zweite Medikament durch eine Einlassseite der zweiten Röhre (220) in den Bevorratungsteil (250) eingeführt und aus dem Bevorratungsteil (250) durch einen Auslass der zweiten Röhre (220) in einer Auslassrichtung ausgelassen wird;
einen Druckteil (300), der auf einer Seite der Abdeckung angeordnet und durch eine Scharnierbewegung bewegbar ist,
wobei der Druckteil (300) eingerichtet ist, um auf den Bevorratungsteil (250) einen Druck aufzubringen, um das zweite Medikament auszulassen, wenn der Druckteil (300) durch die Scharnierbewegung bewegt wird;
ein Rückschlagventil (240), das mit einer Seite des Bevorratungsteils (250) in der Auslassrichtung verbunden ist und mit der Auslassseite der zweiten Röhre (220) verbunden ist, wobei das Rückschlagventil (240) eingerichtet ist, um zu verhindern, dass das zweite Medikament aus dem Bevorratungsteil ausgelassen wird, bevor der Bevorratungsteil vollständig mit dem zweiten Medikament gefüllt wurde, und um zu ermöglichen, dass das zweite Medikament aus dem Bevorratungsteil nur ausgelassen wird,
wenn auf den Bevorratungsteil ein spezifischer Druck durch die Scharnierbewegung des Druckteils (300) aufgebracht wird,
nachdem der Bevorratungsteil (250) vollständig mit dem zweiten Medikament gefüllt wurde; und
einen Regulierungsteil (400) an einer zweiten Seite der unteren Abdeckung (120) gegenüber der ersten Seite der unteren Abdeckung (120), wobei der Regulierungsteil (400) eingerichtet ist, um eine Bewegung des Druckteils (300) zu regeln, indem er die Scharnierbewegung des Druckteils (300) verhindert oder ermöglicht.

2. Medikamenteninfusionsvorrichtung nach Anspruch 1, wobei der Regulierungsteil (400) eine Verriegelungsplatte (410), die an der zweiten Seite der unteren Abdeckung (120) angeordnet ist, um drehbar zu sein, und einen Anschlagstift (413), der sich von einer Seitenfläche der Verriegelungsplatte (410) hin zu der ersten Seite der unteren Abdeckung (120) erstreckt, aufweist,
wobei der Druckteil (300) einen Anschlagteil (330) aufweist, der sich von einer Seite davon hin zu der ersten Seite der unteren Abdeckung (120) erstreckt, und
wobei die Bewegung des Druckteils (300) geregelt wird, während der Anschlagstift (413) gemäß einer Drehrichtung der Verriegelungsplatte (410) mit oder von dem Anschlagteil (330) gekoppelt oder getrennt wird.

3. Medikamenteninfusionsvorrichtung nach Anspruch 2, wobei die Verriegelungsplatte (410) einen Sitzteil (412), der an einem mittigen Abschnitt davon angeordnet ist, und einen Verriegelungsstift (420), der angeordnet ist, um in das und aus dem Sitzteil (412) eingesetzt und extrahiert zu werden, aufweist.

4. Medikamenteninfusionsvorrichtung nach Anspruch 3, wobei die Drehung der Verriegelungsplatte (410) gemäß einer Anordnung des Verriegelungsstifts (420) geregelt wird.

5. Medikamenteninfusionsvorrichtung nach Anspruch 3, wobei eine Drehung der Verriegelungsplatte (410) verhindert wird, wenn der Verriegelungsstift (420) im Inneren des Sitzteils (412) angeordnet ist, und eine Drehung der Verriegelungsplatte (410) ermöglicht wird, wenn der Verriegelungsstift (420) außerhalb des Sitzteils (412) angeordnet ist.

6. Medikamenteninfusionsvorrichtung nach Anspruch 1, ferner umfassend:
ein Freigabeelement (500), das mit dem Regulierungsteil (400) derart gekoppelt ist, dass eine Betätigung des Regulierungsteils (400) durchgeführt wird.

7. Medikamenteninfusionsvorrichtung nach Anspruch 5, ferner umfassend:
ein Freigabeelement (500), das einen Freigabevorsprung (501) aufweist, der in ein in dem Sitzteil (412) gebildetes Durchgangsloch (401) eingesetzt ist,
wobei das Freigabeelement (500) mit dem Regulierungsteil (400) gekoppelt ist und der Verriegelungsstift (420) außerhalb des Sitzteils (412) angeordnet ist, wenn der Freigabevorsprung (501) durch das Durchgangsloch (401) verläuft, um auf den Verriegelungsstift (420) zu drücken,
und
wobei der Verriegelungsstift (420) im Inneren des Sitzteils (412) angeordnet ist, wenn das Freigabeelement (500) von dem Regulierungsteil (400) freigegeben wird.

8. Medikamenteninfusionsvorrichtung nach Anspruch 1, wobei die erste Röhre (210) und die zweite Röhre (220) in der Abdeckung (100) angeordnet sind, sodass sie parallel zueinander sind.

9. Medikamenteninfusionsvorrichtung nach Anspruch 8, ferner umfassend:
eine Durchflussrate-Einstellvorrichtung (230), die in einem oder mehreren Durchgängen der ersten Röhre (210) und der zweiten Röhre (220) installiert ist.

10. Medikamenteninfusionsvorrichtung nach Anspruch 1, ferner umfassend:
einen Befestigungsteil (160), der auf einer Seite der Abdeckung (100) angeordnet ist.

## Revendications

1. Appareil de perfusion de médicament comprenant :
un couvercle (100) ayant un couvercle supérieur (110) et un couvercle inférieur (120);
un premier tube (210) situé à l'intérieur du couvercle (100) et à travers lequel un premier médicament circule;
une partie de stockage (250) située à l'intérieur du couvercle (100) sur un premier côté du couvercle inférieur (120), dans lequel un espace contenant un second médicament est prévu dans un intérieur de la partie de stockage (250),
un second tube (220) relié à des côtés opposés de la partie de stockage (250), de telle sorte que le second médicament est introduit par un côté d'entrée du second tube (220) dans la partie de stockage (250) et déchargé de la partie de stockage (250) par un orifice de sortie du second tube (220) dans une direction de décharge;
une partie de pressage (300) située sur un côté du couvercle et pouvant se déplacer par un mouvement d'articulation, dans lequel la partie de pressage (300) est configurée pour appliquer une pression à la partie de stockage (250) pour décharger le second médicament lorsque la partie de pressage (300) est déplacée par le mouvement d'articulation;
un clapet anti-retour (240) relié à un côté de la partie de stockage (250) dans la direction de décharge et relié au côté de sortie du second tube (220), dans lequel le clapet anti-retour (240) est configuré pour empêcher la décharge du second médicament à partir de la partie de stockage avant le remplissage complet de la partie de stockage avec le second médicament et pour permettre la décharge du second médicament à partir de la partie de stockage seulement lorsqu'une pression déterminée est appliquée à la partie de stockage par le mouvement d'articulation de la partie de pressage (300) après que la partie de stockage (250) est complètement remplie avec le second médicament; et
une partie de régulation (400) au niveau d'un second côté du couvercle inférieur (120) opposé au premier côté du couvercle inférieur (120), dans lequel la partie de régulation (400) est configurée pour réguler le mouvement de la partie de pressage (300) en empêchant ou en permettant le mouvement d'articulation de la partie de pressage (300).

2. Appareil de perfusion de médicament selon la revendication 1, dans lequel la partie de régulation (400) comprend une plaque de verrouillage (410) située au niveau du second côté du couvercle inférieur (120) pour pouvoir tourner, et une tige de butée (413) s'étendant depuis une surface latérale de la plaque de verrouillage (410) vers le premier côté du couvercle inférieur (120),
dans lequel la partie de pressage (300) comprend une partie d'arrêt (330) s'étendant depuis un côté de celle-ci vers le premier côté du couvercle inférieur (120), et
dans lequel le mouvement de la partie de pressage (300) est régulé tandis que la tige de butée (413) est couplée à ou séparée de la partie d'arrêt (330) selon une direction de rotation de la plaque de verrouillage (410).

3. Appareil de perfusion de médicament selon la revendication 2, dans lequel la plaque de verrouillage (410) comprend une partie d'assise (412) située dans une partie centrale de celle-ci, et une goupille de verrouillage (420) située pour être insérée dans et extraite de la partie d'assise (412).

4. Appareil de perfusion de médicament selon la revendication 3, dans lequel la rotation de la plaque de verrouillage (410) est régulée en fonction d'un emplacement de la goupille de verrouillage (420).

5. Appareil de perfusion de médicament selon la revendication 3, dans lequel la rotation de la plaque de verrouillage (410) est empêchée si la goupille de verrouillage (420) est située à l'intérieur de la partie d'assise (412), et la rotation de la plaque de verrouillage (410) est autorisée si la goupille de verrouillage (420) est située à l'extérieur de la partie d'assise (412).

6. Appareil de perfusion de médicament selon la revendication 1, comprenant en outre :
un élément de libération (500) couplé à la partie de régulation (400), de telle sorte qu'une opération de la partie de régulation (400) est effectuée.

7. Appareil de perfusion de médicament selon la revendication 5, comprenant en outre :
un élément de libération (500) comprenant un bossage de libération (501) qui est inséré dans un trou traversant (401) formé dans la partie d'assise (412),
dans lequel l'élément de libération (500) est couplé à la partie de régulation (400), et la goupille de verrouillage (420) est située à l'extérieur de la partie d'assise (412) si le bossage de libération (501) passe à travers le trou traversant (401) pour appuyer sur la goupille de verrouillage (420), et
dans lequel la goupille de verrouillage (420) est située à l'intérieur de la partie d'assise (412) si l'élément de libération (500) est libéré de la partie de régulation (400) .

8. Appareil de perfusion de médicament selon la revendication 1, dans lequel le premier tube (210) et le second tube (220) sont situés dans le couvercle (100) pour être linéairement parallèles l'un à l'autre.

9. Appareil de perfusion de médicament selon la revendication 8, comprenant en outre :
un appareil de réglage de débit (230) installé dans un ou plusieurs passages du premier tube (210) et du second tube (220) .

10. Appareil de perfusion de médicament selon la revendication 1, comprenant en outre :
une partie de fixation (160) située sur un côté du couvercle (100) .
